# EUROPEAN PATENT APPLICATION

(11) **EP 4 775 581 A2**
(43) Date of publication of application: **15.07.2026**
(21) Application number: 25225068.3
(22) Date of filing: 18.12.2025
(51) Int. Cl.: C07D 471/04

(54) **PROCESS FOR PREPARING FINERENONE**

(30) Priority: 14.01.2025 SI 202500004
(71) Applicant: KRKA, D.D., Novo Mesto, 8501 Novo mesto (SI)
(72) Inventor: SLAPSAK, Dejan, 8501 Novo mesto (SI); BEZENSEK, Jure, 8501 Novo mesto (SI)
(74) Representative: Kutzenberger Wolff & Partner

(57) **Abstract**

The present invention relates to a process for preparing finerenone. In addition, the invention relates to finerenone hemihydrate and its solid form.

## Description

Priority is claimed of Slovenian patent application no. P-202500004 that was filed on 14 January 2025.

### Technical Field

The present invention relates to a process for preparing finerenone. Additionally, the invention relates to finerenone hemihydrate and its solid-state form.

### Background of the Invention

Finerenone, (4S)-4-(4-cyano-2-methoxyphenyl)-5-ethoxy-2,8-dimethyl-1,4-dihydro-1,6-naph-thyridine-3-carboxamide, is a non-steroidal mineralocorticoid receptor antagonist and can be used as an agent for the prevention and/or treatment of cardiovascular and renal disorders, such as heart failure and diabetic nephropathy.

Several processes for preparing the compound of formula I are known.

According to the processes described in applications WO 2008/104306 A1 and WO 2016/016287 A1, expensive column chromatography was used to separate the racemic mixture into individual enantiomers.

Improved processes have been developed for preparing enantiomers of the final product or intermediates by resolving racemic mixtures using solutions of chiral acids. Processes using chiral substituted tartaric acid esters have been described in applications WO 2019/206909 A1, WO 2021/074072 A1, WO 2021/074078 A1, WO 2021/074079 A1, CN 115340539 A, CN 115340540 A, and CN 118027027 A.

Application CN118479984 discloses processes for resolving racemic mixtures in which a tartaric acid derivative is used as the resolving agent, and the processes can be carried out under mild conditions.

However, there remains a need for an improved process for preparing finerenone suitable for industrial production, which overcomes certain drawbacks of known processes.

### Detailed Description of the Invention

A first aspect of the invention relates to a process for preparing finerenone, a compound of formula I 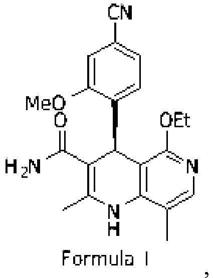 which comprises deprotecting the compound of formula II 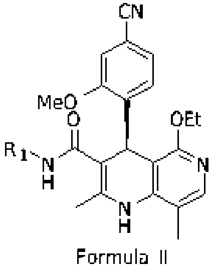 wherein
R1 is selected from
(i) C₁-C₆ alkyl,
(ii) phenyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group, and
(ii) benzyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group;
in the presence of trifluoroacetic acid and/or trifluoromethanesulfonic acid, preferably at elevated temperature, preferably followed by the addition of an antisolvent to obtain a precipitate.

The process for preparing finerenone according to the invention comprises the step:
a) mixing a compound of formula II with trifluoroacetic acid and/or trifluoromethanesulfonic acid; preferably with both, trifluoroacetic acid and trifluoromethanesulfonic acid.

In the compound of formula II, R1 is selected from
(i) C₁-C₆ alkyl; preferably tert-butyl;
(ii) phenyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group; preferably phenyl; and
(iii) benzyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group; preferably p-methoxybenzyl, 2,4-dimethoxybenzyl, or 3,4-dimethylbenzyl.

Preferably, R1 is selected from the group consisting of
(i) tert-butyl,
(ii) phenyl,
(iii) p-methoxybenzyl, 2,4-dimethoxybenzyl, and 3,4-dimethylbenzyl.

More preferably, the compound of formula II is the compound of formula II-a

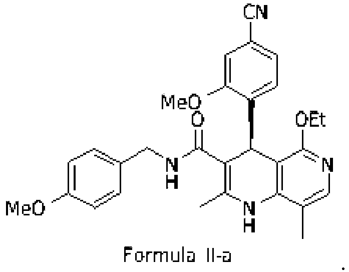

Preferably, deprotection of the compound of formula II or the compound of formula II-a is carried out in a mixture with both, trifluoroacetic acid and trifluoromethanesulfonic acid.

In preferred embodiments, the deprotection of the compound of formula II or the compound of formula II-a is carried out in a solvent. When a solvent is used, it is preferably selected from dichloromethane, tetrahydrofuran, acetonitrile, N-methylpyrrolidone, and N,N-dimethylacetamide.

In other preferred embodiments, the deprotection of the compound of formula II or the compound of formula II-a is carried out in the absence of a solvent. The deprotection without the addition of a solvent, i.e. in the absence of solvent, is particularly preferred. The compound of formula II or the compound of formula II-a is then preferably dissolved or dispersed in trifluoroacetic acid and/or trifluoromethanesulfonic acid.

Preferably, the deprotection is carried out at a temperature from 40 °C to 75 °C, preferably from 45 °C to 70 °C, more preferably from 50 °C to 65 °C, and most preferably from 50 °C to 60 °C. Optionally, the deprotection reaction is carried out under a nitrogen atmosphere. It is preferred that the presence of water in the reaction mixture is minimized.

Preferably, the molar ratio of the compound of formula II or formula II-a to trifluoromethanesulfonic acid is from 1:0.2 to 1:3, preferably from 1:0.2 to 1:2, more preferably from 1:0.3 to 1:1.5, even more preferably from 1:0.3 to 1:1, and most preferably about 1:0.5.

Preferably, the molar ratio of the compound of formula II or formula II-a to trifluoroacetic acid is from 1:10 to 1:30, preferably from 1:15 to 1:25, more preferably about 1:20.

Preferably, the molar ratio of the compound of formula II or formula II-a to the sum of the molar amount of trifluoromethanesulfonic acid and trifluoroacetic acid is 1:0.2-2:10-30, preferably 1:0.3-1:15-25, more preferably 1:0.5:20.

Preferably, after completion of the deprotection reaction, an antisolvent is added to the reaction mixture, preferably selected from C₁-C₆ alcohols, ethyl acetate, or mixtures thereof, more preferably a mixture of ethanol and ethyl acetate.

Preferably, the weight ratio of ethanol to ethyl acetate is from 1:1 to 1:10, preferably from 1:2 to 1:8, more preferably from 1:3 to 1:6, even more preferably from 1:3 to 1:5, and most preferably about 1:4.

After preferred addition of the antisolvent, the reaction mixture is preferably cooled, preferably to a temperature from 0 °C to 10 °C, preferably to about 5 °C, to obtain a precipitate.

The thus obtained precipitate is preferably filtered. Filtration may be carried out by any known method, e.g., standard filters or glass fiber filters.

By introducing the precipitation step, evaporation of highly corrosive acids, as required in the process described in application CN 118027027 A, is not necessary, and consequently the risk of equipment damage is reduced.

Preferably, the obtained precipitate is subsequently dissolved/suspended in a solvent, preferably in a mixture of a C₁-C₆ alcohol and water, more preferably in a mixture of methanol or ethanol and water, and most preferably in a mixture of methanol and water.

Preferably, the weight ratio of methanol to water is from 1:0.5 to 1:10, preferably from 1:1 to 1:5, more preferably from 1:1 to 1:4, and most preferably about 1:2.5.

Preferably, the pH of the solution/suspension is adjusted to a value of 8 to 10, preferably 8 to 9, preferably by adding a base selected from NaHCO₃, Na₂CO₃, Na₃PO₄, Na₂HPO₄, NaH₂PO₄, NH₃, NaOH, LiOH, KOH, (NH₄)₂CO₃, Li₂CO₃, K₂CO₃, or K₃PO₄, preferably NaHCO₃.

The base may be added in solid form or as a solution, preferably as a solution.

Prior to the addition of the base, the suspension is preferably cooled, preferably to a temperature from 0 °C to 10 °C, preferably to about 5 °C.

Optionally, the precipitate may be filtered by any known filtration method, e.g., using standard filters or glass fiber filters.

The obtained finerenone may be recrystallized from a C₁-C₆ alcohol, preferably ethanol.

After recrystallization, the finerenone may be filtered and dried.

A preferred embodiment of the present invention is a process for preparing finerenone, a compound of formula I 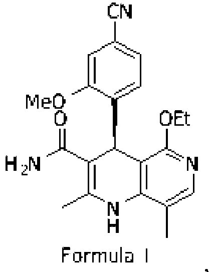 comprising the following steps:
a) mixing the compound of formula II 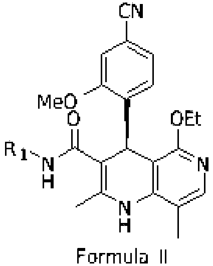 with trifluoroacetic acid and/or trifluoromethanesulfonic acid; preferably trifluoroacetic acid and trifluoromethanesulfonic acid;
b) heating the reaction mixture obtained in step a), preferably to a temperature from 40 °C to 75 °C, preferably from 45 °C to 70 °C, more preferably from 50 °C to 65 °C, and most preferably from 50 °C to 60 °C;
c) adding an antisolvent; and
d) cooling, preferably to a temperature from 0 °C to 10 °C, preferably to about 5 °C, to obtain a precipitate;
wherein R1 is as defined above.

In preferred embodiments, the process additionally comprises the steps:
e) filtering off the precipitate obtained in step d);
f) dissolving or suspending the precipitate obtained in step e) in a solvent;
g) cooling, preferably to a temperature from 0 °C to 10 °C, preferably to about 5 °C;
h) adjusting the pH to 8 to 10, preferably to 8 to 9, to obtain a precipitate;
i) optionally, filtering the precipitate obtained in step h); and
j) optionally, recrystallizing the compound of formula I from a C₁-C₆ alcohol, preferably ethanol;
wherein R1 is as defined above.

In preferred embodiments, the process for preparing finerenone, a compound of formula I 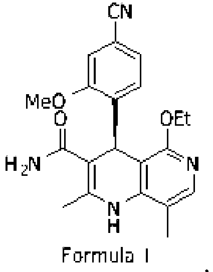 comprises the following steps:
a) mixing the compound of formula II-a 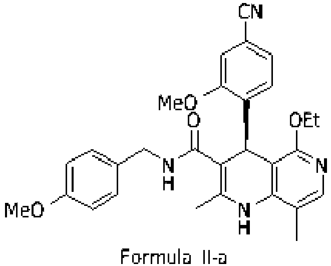 with trifluoroacetic acid and/or trifluoromethanesulfonic acid; preferably trifluoroacetic acid and trifluoromethanesulfonic acid;
b) heating the reaction mixture obtained in step a), preferably to a temperature from 40 °C to 75 °C, preferably from 45 °C to 70 °C, more preferably from 50 °C to 65 °C, and most preferably from 50°C to 60 °C;
c) adding an antisolvent;
d) cooling, preferably to a temperature from 0 °C to 10 °C, optionally to about 5 °C, to obtain a precipitate;
e) filtering off the precipitate obtained in step d);
f) dissolving or suspending the precipitate obtained in step e) in a solvent;
g) cooling, preferably to a temperature from 0 °C to 10 °C, preferably to about 5 °C;
h) adjusting the pH to 8 to 10, preferably to 8 to 9, to obtain a precipitate;
i) optionally, filtering the precipitate obtained in step h);
j) optionally, recrystallizing the compound of formula I from a C₁-C₆ alcohol, preferably ethanol.

In preferred embodiments, the process for preparing finerenone, a compound of formula I 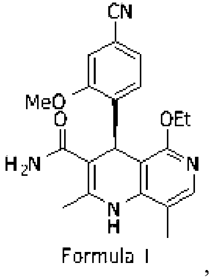 comprises the following steps:
a) mixing the compound of formula II 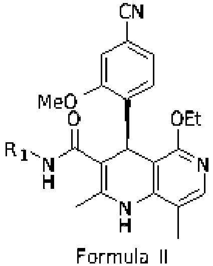 with trifluoroacetic acid and/or trifluoromethanesulfonic acid; preferably trifluoroacetic acid and trifluoromethanesulfonic acid;
b) heating the reaction mixture obtained in step a), preferably to a temperature from 40 °C to 75 °C, preferably from 45 °C to 70 °C, more preferably from 50 °C to 65 °C, and most preferably from 50°C to 60 °C;
c) adding an antisolvent;
d) cooling, preferably to a temperature from 0 °C to 10 °C, preferably to about 5 °C, to obtain a precipitate;
e) filtering off the precipitate obtained in step d);
f) dissolving or suspending the precipitate obtained in step e) in a solvent;
g) cooling, preferably to a temperature from 0 °C to 10 °C, preferably to about 5 °C;
h) adjusting the pH to 8 to 10, preferably to 8 to 9, to obtain a precipitate;
i) optionally, filtering the precipitate obtained in step h);
j) optionally, recrystallizing the compound of formula I from a C₁-C₆ alcohol, preferably ethanol;

wherein R1 is as defined above; and
wherein one or more, preferably all, of the following conditions are met:
   - the molar ratio of the compound of formula II to trifluoromethanesulfonic acid is from 1:0.3 to 1:1.5, preferably from 1:0.3 to 1:1, more preferably about 1:0.5;
   - the molar ratio of the compound of formula II to trifluoroacetic acid is from 1:15 to 1:25, preferably about 1:20;
   - the molar ratio among the compound of formula II, trifluoromethanesulfonic acid, and trifluoroacetic acid is 1:0.3-1:15-25, preferably 1:0.5:20;
   - the antisolvent in step c) is a mixture of ethanol and ethyl acetate, preferably in a ratio from 1:3 to 1:6, more preferably about 1:4;
   - the solvent in step f) is a mixture of methanol and water, preferably in a ratio from 1:1 to 1:4, most preferably about 1:2.5;
   - in step h), the pH of the suspension is adjusted by adding a base, preferably NaHCO₃, more preferably an aqueous solution of NaHCO₃.

In preferred embodiments, the process for preparing finerenone, a compound of formula I 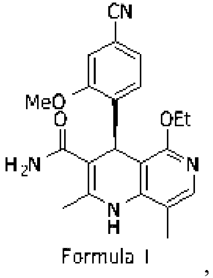 comprises the following steps:
a) mixing the compound of formula II-a 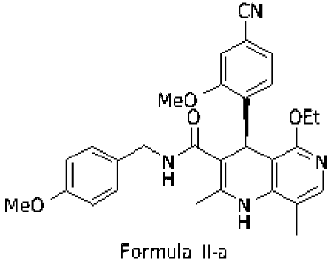 with trifluoroacetic acid and/or trifluoromethanesulfonic acid; preferably trifluoroacetic acid and trifluoromethanesulfonic acid;
b) heating the reaction mixture obtained in step a), preferably to a temperature from 40 °C to 75 °C, preferably from 45 °C to 70 °C, more preferably from 50 °C to 65 °C, and most preferably from 50 °C to 60 °C;
c) adding an antisolvent;
d) cooling, preferably to a temperature from 0 °C to 10 °C, optionally to about 5 °C, to obtain a precipitate;
e) filtering off the precipitate obtained in step d);
f) dissolving or suspending the precipitate obtained in step e) in a solvent;
g) cooling, preferably to a temperature from 0 °C to 10 °C, preferably to about 5 °C;
h) adjusting the pH to 8 to 10, preferably to 8 to 9, to obtain a precipitate;
i) optionally, filtering the precipitate obtained in step h);
j) optionally, recrystallizing the compound of formula I from a C₁-C₆ alcohol, preferably ethanol;
wherein one or more, preferably all, of the following conditions are met:
- the molar ratio of the compound of formula II-a to trifluoromethanesulfonic acid is from 1:0.3 to 1:1.5, preferably from 1:0.3 to 1:1, more preferably about 1:0.5;
- the molar ratio of the compound of formula II-a to trifluoroacetic acid is from 1:15 to 1:25, preferably about 1:20;
- the molar ratio among the compound of formula II-a, trifluoromethanesulfonic acid, and trifluoroacetic acid is 1:0.3-1:15-25, preferably 1:0.5:20;
- the antisolvent in step c) is a mixture of ethanol and ethyl acetate, preferably in a ratio from 1:3 to 1:6, more preferably about 1:4;
- the solvent in step f) is a mixture of methanol and water, preferably in a ratio from 1:1 to 1:4, most preferably about 1:2.5;
- in step h), the pH of the suspension is adjusted by adding a base, preferably NaHCO₃, more preferably an aqueous solution of NaHCO₃.

Preferably, the precipitate obtained in step d) contains finerenone hemihydrate.

Without wishing to be bound to any scientific theory, the hemihydrate form is obtained due to trace water present in the system, which may originate e.g. from residual moisture in starting materials, hygroscopic acids, solvents, atmospheric exposure, or equipment surfaces. If necessary, the required amount of water can be added.

Finerenone hemihydrate was identified in the precipitate by dynamic vapor sorption (DVS) analysis.

DVS analysis may be performed using a dynamic vapor sorption system (SPS-1u), ProUmid GmbH. The sample may be subjected to a humidity program of 40-0-80-0% RH at 25 °C with 10% RH steps. The sample weight may be measured every 10 minutes.

The compound of formula II or the compound of formula II-a may be prepared by any process known in the art, e.g., CN 118027027 A.

The compound of formula II may be prepared (i.e. the enantiomer of formula II may be purified) by the following process:
A) mixing the compound of formula IV 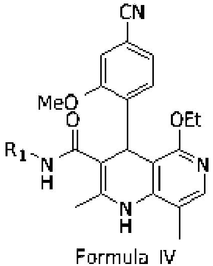 with the compound of formula Va or the compound of formula Vb 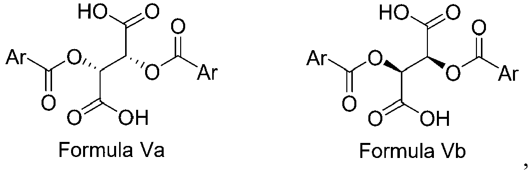 or a hydrate thereof, to obtain the compound of formula III-1a or the compound of formula III-1b or the compound of formula III-1c or the compound of formula III-1d 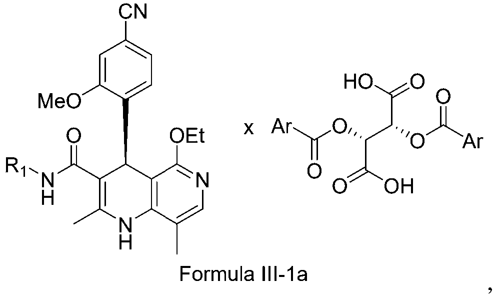 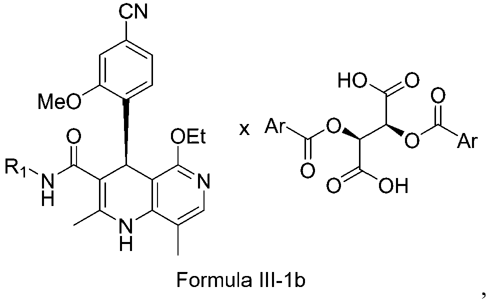 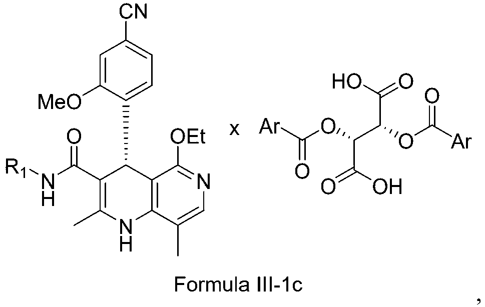 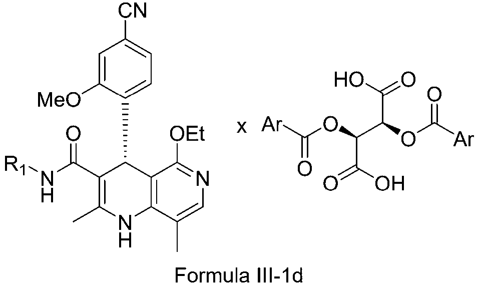
B) optionally, treating the compound of formula III-1a or the compound of formula III-1b with a base to obtain the compound of formula II 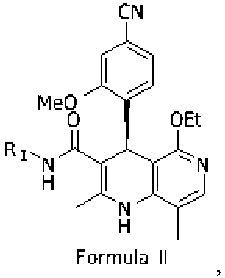 wherein
   R1 is as defined above, and
   Ar is benzyl unsubstituted or substituted with a substituent selected from methoxy, ethoxy, halogen, hydroxyl, nitro, cyano, methyl, or ethyl group;
      and/or
C) mixing the compound of formula IV 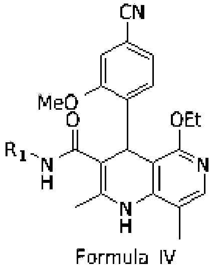 with the compound of formula VIa or the compound of formula VIb 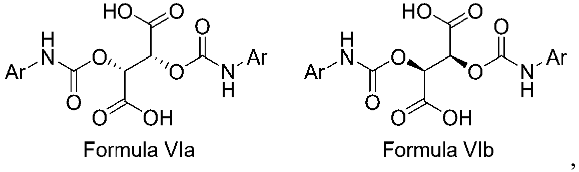 or a hydrate thereof, to obtain the compound of formula III-2a or the compound of formula III-2b or the compound of formula III-2c or the compound of formula III-2d 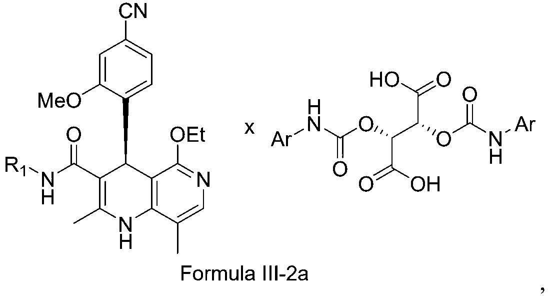 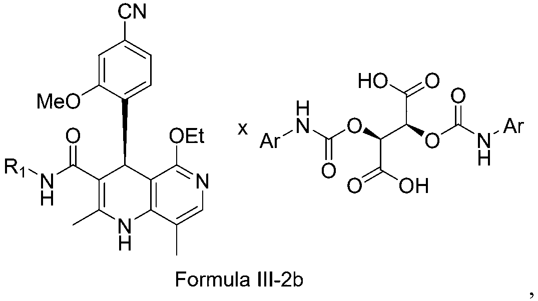 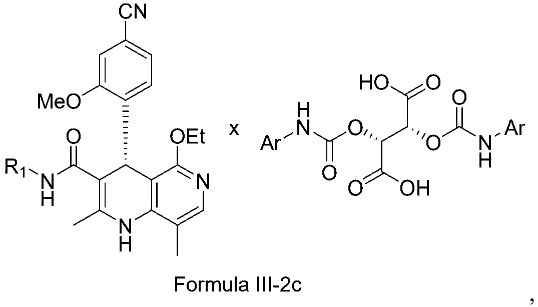 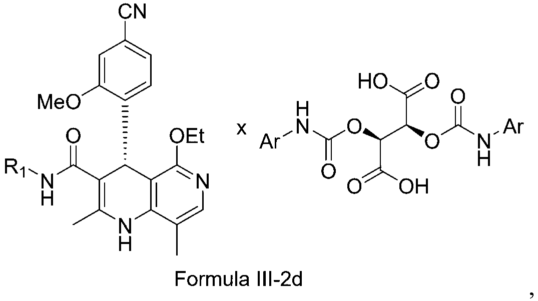
D) optionally, treating the compound of formula III-2a or the compound of formula III-2b with a base to obtain the compound of formula II 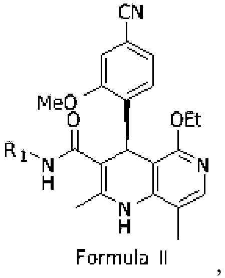 wherein
   R1 is as defined above, and
   Ar is benzyl unsubstituted or substituted with a substituent selected from methoxy, ethoxy, halogen, hydroxyl, nitro, cyano, methyl, or ethyl group.

Preferably, the halogen is selected from Cl and Br, more preferably Cl.

The compound of formula VIa/b wherein Ar is p-toluoyl is known as O,O'-di-p-toluoyl-tartaric acid (DTTA).

Above steps A) and B) involve a separation of enantiomers, whereas above steps C) and D) likewise involve a separation of enantiomers. When the compound of formula IV is provided as a racemate, above steps A) and optional B) involve a racemic resolution. When the compound of formula IV is provided in enantiomeric excess of one enantiomer, e.g. resulting from enantioselective synthesis, above steps A) and optional B) involve increasing the enantiomeric excess.

Steps B) and D) are independently of one another optional, because they are only needed when for the purpose of subsequent uses, the thus formed compound (adduct, acid addition salt, aggregate, or the like) of any one of formulas III-1a, III-1b, III -1c or III-1d needs to be separated from the compound of formula Va and the compound of formula Vb, respectively, which in turn may also be reused.

Above step A) involves mixing the compound of formula IV with either the compound of formula Va (one of two enantiomers), or with the compound of formula Vb (other of two enantiomers).

The compound of formula IV is preferably provided as a racemate, i.e. an equimolar mixture of the following two enantiomers:

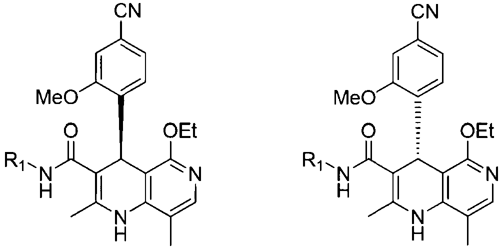

It is contemplated, however, that the compound of formula IV is provided with an enantiomeric excess of one enantiomer, e.g. resulting from enantioselective synthesis.

When mixing the racemic or enantiomerically enriched compound of formula IV with the compound of formula Va (one of two enantiomers) 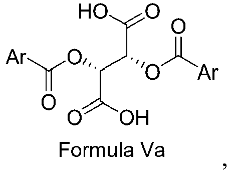 formation of the compound of formula III-1a or the compound of formula III-1c 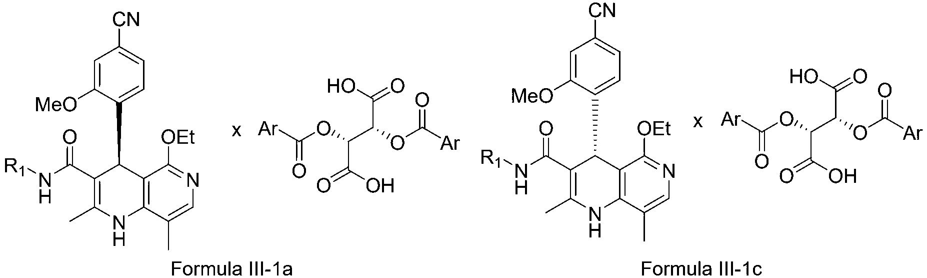 is possible. As compounds III-1a and III-1c have different properties, e.g. different solubility in a given solvent system, the two enantiomers of the compound of formula IV may be separated from one another based upon these different properties (e.g., diastereomeric crystallization). For example, one of the two enantiomers of the compound of formula IV may stay in solution, whereas the other of the two enantiomers of the compound of formula IV may form together with the compound of formula Va (one of two enantiomers) compound III-1a or III-1c and precipitate. The two enantiomers of the compound of formula IV may then easily be separated from one another, e.g. by filtration. Either the filtrate or the precipitate may subsequently be used.

When mixing the racemic or enantiomerically enriched mixture of the compound of formula IV with the compound of formula Vb (other of two enantiomers) 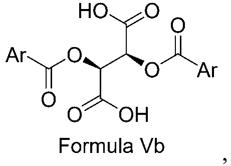 formation of the compound of formula III-1b or the compound of formula III-1d 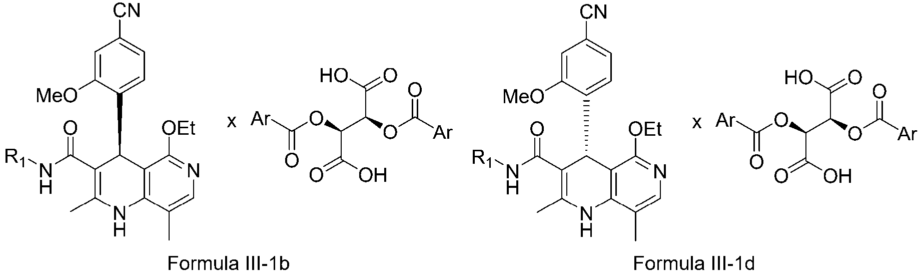 is possible. As compounds III-1b and III-1d have different properties, e.g. different solubility in a given solvent system, the two enantiomers of the compound of formula IV may be separated from one another based upon these different properties (e.g., diastereomeric crystallization). For example, one of the two enantiomers of the compound of formula IV may stay in solution, whereas the other of the two enantiomers of the compound of formula IV may form together with the compound of formula Vb (other of two enantiomers) compound III-1b or III-1d and precipitate. The two enantiomers of the compound of formula IV may then easily be separated from one another, e.g. by filtration. Either the filtrate or the precipitate may subsequently be used.

The same principle of separation of enantiomers applies to step C), i.e., mixing the compound of formula IV, which as a consequence of preceding steps A.) and optional B.) may be provided in form of an enriched enantiomer, i.e. an enantiomeric excess, with either the compound of formula VIa (one of two enantiomers) or the compound of formula VIb (other of two enantiomers).

Thus, the enantiomers (R and S) of the compound of general formula IV or more specifically, formula IV-a, can be separated from one another using one enantiomer of chiral acids of general formula Va and Vb, and alternatively or additionally, one enantiomer of chiral acids of general formula VIa and VIb, respectively. All combinations of enantiomers R and S together with chiral acids Va, Vb, VIa and VIb lead to separation to some degree, therefore all combinations of reagents are reasonable and contemplated according to the invention.

The pure enantiomers of chiral acids of general formula Va and Vb, and VIa and VIb, are commercially available.

The compound of formula II-a may be prepared (i.e. the enantiomer of formula II-a may be purified) in analogy by the following process:
(1) mixing the compound of formula IV-a 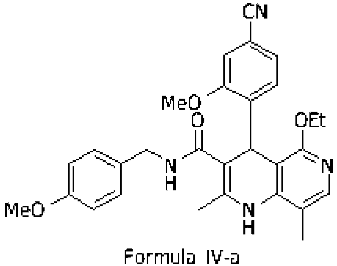 with the compound of formula Va or the compound of formula Vb 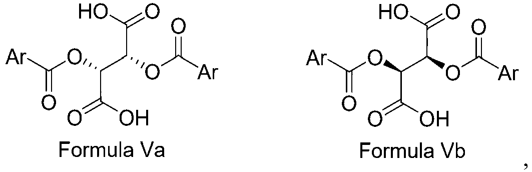 or a hydrate thereof, to obtain the compound of formula III-a-1a or the compound of formula III-a-1b or the compound of formula III-a-1c or the compound of formula III-a-1d: 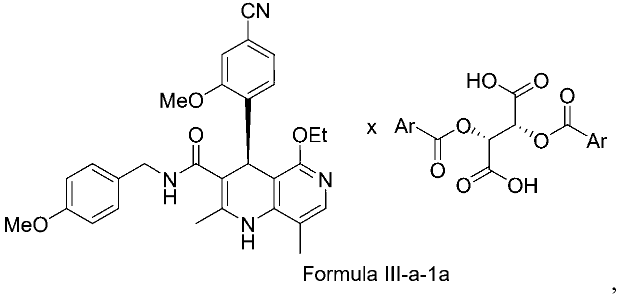 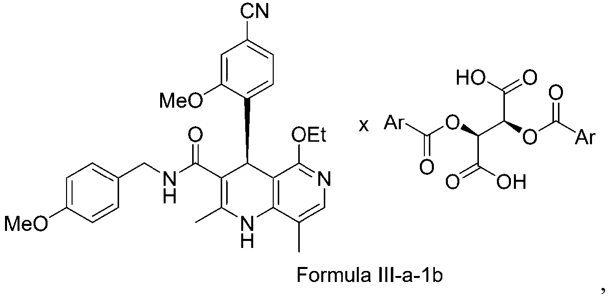 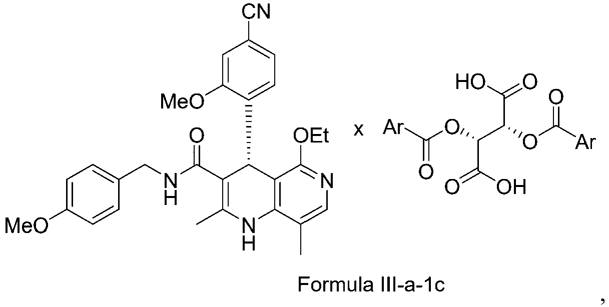 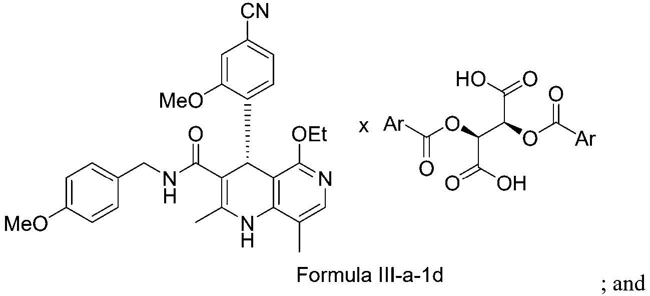
(2) optionally, treating the compound of formula III-a-1a or the compound of formula III-a-1b with a base to obtain the compound of formula II-a 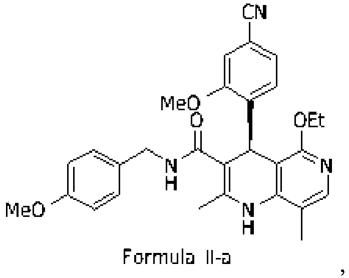 wherein Ar is as defined above.

Preferably, the solvent in step (1) or step (2) is selected from methanol, ethanol, water, tetrahydrofuran, 2-methyltetrahydrofuran, dioxane, or mixtures thereof, preferably from methanol, ethanol, water, or mixtures thereof, more preferably from ethanol, water, or a mixture thereof.

Preferably, the base in step (2) is selected from NaHCO₃, Na₂CO₃, Na₃PO₄, Na₂HPO₄, NaH₂PO₄, (NH₄)₂CO₃, K₂CO₃, K₃PO₄, or mixtures thereof.

Alternatively or additionally, the compound of formula II-a may be prepared (i.e. the enantiomer of formula II-a may be purified) in analogy by the following process:
(3) mixing the compound of formula IV-a 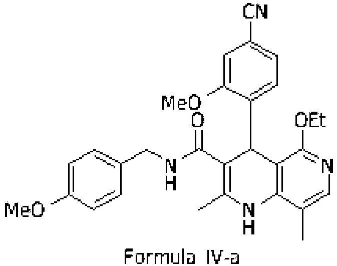 with the compound of formula VIa or the compound of formula VIb 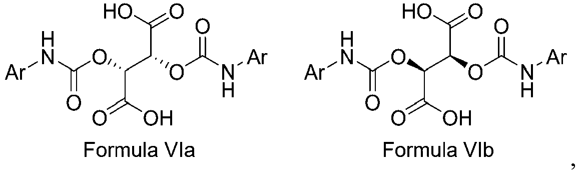 or a hydrate thereof, to obtain the compound of formula III-a-2a or the compound of formula III-a-2b or the compound of formula III-a-2c or the compound of formula III-a-2d: 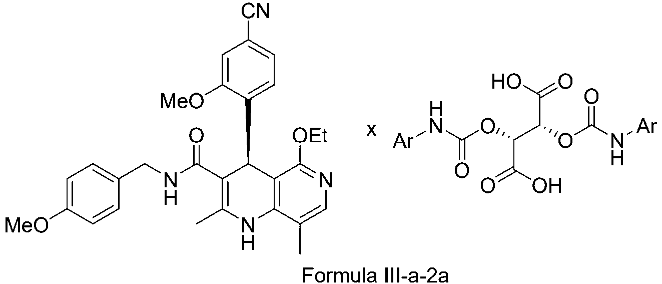 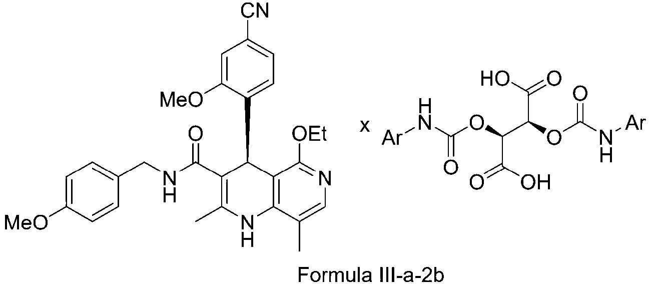 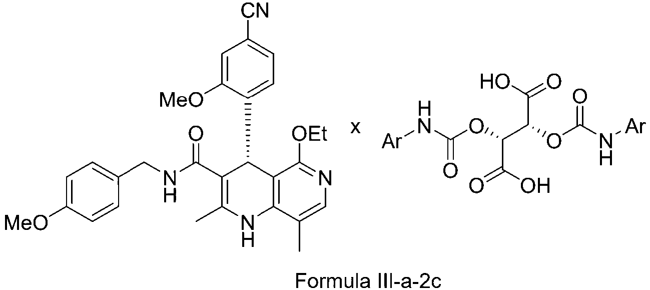 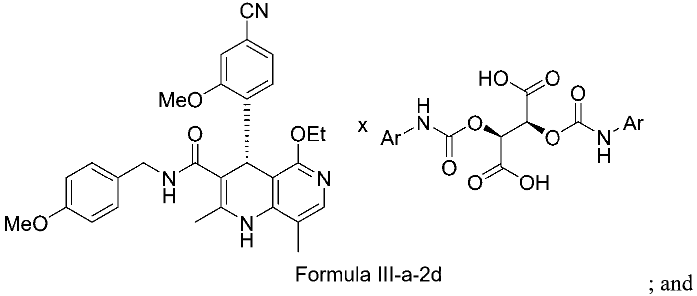
(4) optionally, treating the compound of formula III-a-2a or the compound of formula III-a-2b with a base to obtain the compound of formula II-a 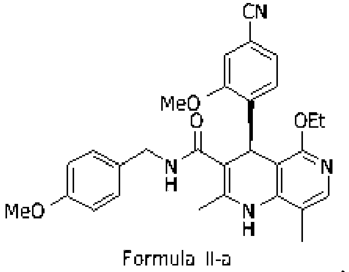 wherein Ar is as defined above.

Preferably, the solvent in step (3) or step (4) is selected from methanol, ethanol, water, tetrahydrofuran, 2-methyltetrahydrofuran, dioxane, or mixtures thereof, preferably from methanol, ethanol, water, or mixtures thereof, more preferably from ethanol, water, or a mixture thereof.

Preferably, the base in step (4) is selected from NaHCO₃, Na₂CO₃, Na₃PO₄, Na₂HPO₄, NaH₂PO₄, (NH₄)₂CO₃, K₂CO₃, K₃PO₄, or mixtures thereof.

The compounds of formula IV and formula IV-a may be prepared by any process known in the art, e.g., the processes described in application CN 118027027 A.

Another aspect of the invention relates to finerenone hemihydrate.

Preferably, finerenone hemihydrate is solid, more preferably crystalline.

Preferably, the solid-state form of finerenone hemihydrate is characterized by an X-ray powder diffraction (XRPD) pattern having at least 5 characteristic peaks selected from 4.3° ± 0.2°, 7.2° ± 0.2°, 8.6° ± 0.2°, 11.4° ± 0.2°, 12.1° ± 0.2°, 17.4° ± 0.2°, 22.4° ± 0.2°, and 26.2° ± 0.2° 2Theta; preferably having all these characteristic peaks.

XRPD patterns may be recorded using an X-ray powder diffractometer (PANalytical X'Pert PRO MPD, Almelo, NL) with Cu Kα radiation (λ = 1.5418 Å) at 45 kV and 40 mA and an X'Celerator detector. The measurement may be performed over a 2Theta range of 3° to 32.5°, with a step size of 0.033° and an integration time of 500 s.

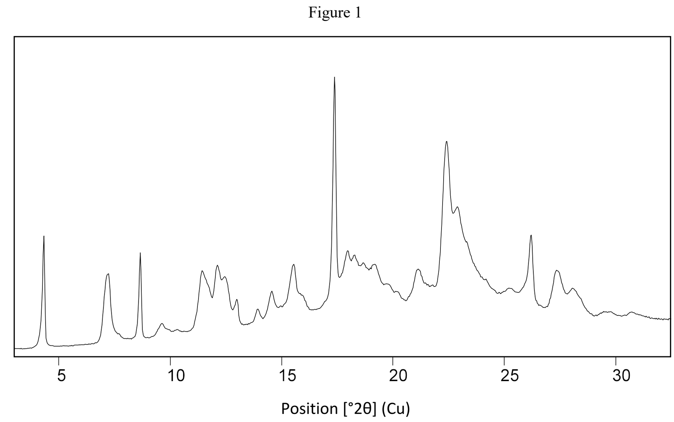

Figure 1 shows the X-ray powder diffraction pattern of finerenone hemihydrate.

Another aspect of the invention relates to the use of the resolving agent compound of formula VI for resolving the racemic mixture of the compound of formula VII:

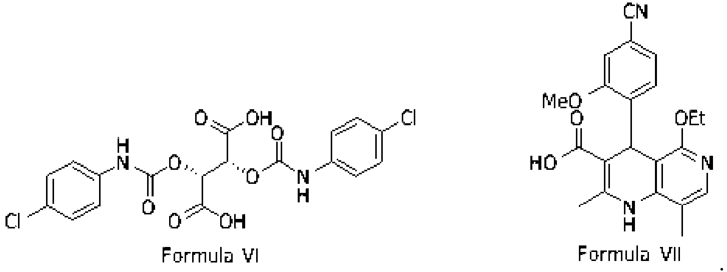

Another aspect of the invention relates to a process for preparing the compound of formula V 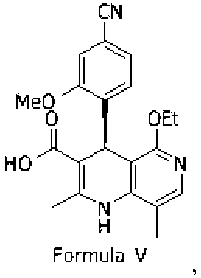 comprising the following steps:
(i) adding the compound of formula VI to the compound of formula VII to form the salt of formula VIII 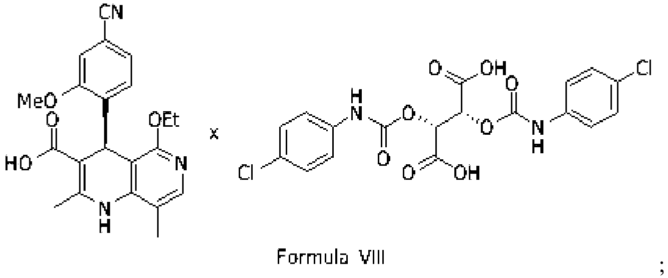
(ii) filtering the resulting precipitate; and
(iii) neutralizing the salt by adding a base to obtain the compound of formula V.

Another aspect of the invention also relates to a process for preparing the compound of formula V 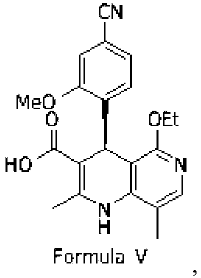 comprising the following steps:
(I) adding the compound of formula VI to a solution of the compound of formula VII;
(II) heating the mixture obtained in step a), preferably to a temperature from 30 °C to 70 °C, preferably from 40 °C to 60 °C, and most preferably from 45 °C to 55 °C;
(III) mixing the heated mixture;
(IV) cooling, preferably to a temperature from 0 °C to 10 °C, preferably to about 5 °C, to obtain the precipitate of formula VIII 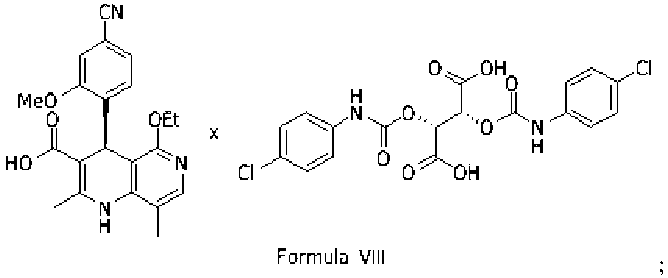
(V) filtering off the resulting precipitate;
(VI) dissolving or suspending the precipitate obtained in step e) in a solvent;
(VII) adjusting the pH to 5 to 8, preferably to 6 to 7;
(VIII) optionally, separating the organic phase from the aqueous phase;
(IX) evaporating the organic solvent; and
(X) optionally, converting the compound of formula V into the compound of formula I.

Preferably, the solvent used in step (I) is a mixture of water and an organic solvent selected from C₁-C₆ alcohols, acetone, or THF, preferably a mixture of water with ethanol, acetone, or THF.

Preferably, the volume ratio of water to ethanol may range from 2:1 to 1:5, preferably from 1:1 to 1:3, and most preferably about 1:2.

Preferably, the volume ratio of water to THF may range from 2:1 to 1:10, preferably from 1:1 to 1:5, and most preferably about 1:3.

Preferably, the volume ratio of water to acetone may range from 2:1 to 1:5, preferably from 1:1 to 1:3, and most preferably about 1:2.

Preferably, the molar ratio of the compound of formula VII to the compound of formula VI is from 1:1 to 1:2, preferably from 1:1 to 1:1.5, and most preferably about 1:1.

Preferably, mixing in step (III) may last from 12 to 24 hours, preferably from 16 to 20 hours, and more preferably about 18 hours.

Preferably, the solvent used in step (VI) is a mixture of water and an organic solvent selected from the group consisting of dichloromethane, ethyl acetate, isopropyl acetate, methyl tert-butyl ether, 1,2-dichloroethane, and chloroform, preferably a mixture of water and dichloromethane.

The volume ratio of dichloromethane to water may range from 30:1 to 10:1, preferably from 25:1 to 15:1, and most preferably about 20:1.

Preferably, in step (VII), the pH may be adjusted by adding a base selected from NaHCO₃, Na₂CO₃, Na₃PO₄, Na₂HPO₄, NaH₂PO₄, NH₃, NaOH, LiOH, KOH, (NH₄)₂CO₃, Li₂CO₃, K₂CO₃, or K₃PO₄, preferably Na₃PO₄. The base may be added in solid form or as a solution, preferably as a solution.

Step (VIII) is preferably performed by using an organic solvent immiscible with water.

In step (X) any known method can be used for converting the compound of formula V into the compound of formula I, i.e., introducing an amino group, for example, the methods described in WO 2021/074078 A1, CN 115340539 A, and CN 115340540 A.

The compound of formula VII can be prepared by any known method, for example, by the method described in WO 2016/016287 A1.

### Examples

The following examples further illustrate the invention but should not be construed as limiting its scope.

### Example 1

In a dry flask purged with nitrogen, 3.0 g of the compound of formula II, 18 g of trifluoroacetic acid, and 0.45 g of trifluoromethanesulfonic acid were mixed. The resulting mixture was heated under a nitrogen atmosphere to 55 °C and maintained at this temperature. The reaction mixture was cooled to room temperature, and 3 ml of ethanol and 12 ml of ethyl acetate were added. The resulting suspension was cooled to 5 °C and stirred for 1 hour. The precipitate obtained was filtered.

### Example 2

The precipitate obtained in Example 1 was analyzed by dynamic vapor sorption (DVS) and X-ray powder diffraction (XRPD).

The analysis was performed using a water vapor sorption system (SPS-1u), ProUmid GmbH. Approximately 85 mg of the sample was subjected to a humidity program of 40-0-80-0% RH at 25 °C with 10% RH steps. Equilibrium was reached at all RH levels except at 0% RH at the end of the first desorption cycle. The sample mass was recorded every 10 minutes.

The DVS analysis showed a 2.4% mass loss, confirming the presence of finerenone hemihydrate.

The X-ray diffractogram was recorded using an X-ray powder diffractometer (PANalytical X'Pert PRO MPD, Almelo, NL) with Cu Kα radiation (λ = 1.5418 Å) at 45 kV and 40 mA and an X' Celerator detector. Measurements were performed in the 2Theta range from 3° to 32.5°, with a step size of 0.033° and an integration time of 500 s. The XRPD pattern is shown in Figure 1.

### Example 3

The precipitate obtained in Example 1 was transferred to another flask, and 6 ml of methanol and 15 ml of water were added. The suspension was cooled to 5 °C, and the pH was adjusted to 8-9 using an 8% NaHCO₃ solution. The precipitate was filtered off and transferred to a fresh flask, where 3 ml of methanol and 4.5 ml of water were added. The suspension was heated under reflux for 2 hours, then cooled to 5 °C and stirred for 1 hour. The precipitate was filtered off and transferred to a clean flask, where 1.8 ml of ethanol was added, and the mixture was heated under reflux for 2 hours. The mixture was cooled to 5 °C, and the precipitate was filtered off. 0.270 g (12%) of the compound of formula I was obtained.

### Example 4

In a flask, 6.0 g of the compound of formula VII was combined with 225 ml of ethanol, 75 ml of water, and 7.23 g of the compound of formula VI. The resulting mixture was heated to 50 °C and stirred at this temperature for 18 hours. The mixture was then cooled to 5 °C and stirred for 4 hours at this temperature. The resulting suspension was filtered, and the precipitate was washed with 6 ml of an EtOH:H₂O mixture (3:1). 4.10 g (31.0%) of the compound of formula VIII was obtained.

The precipitate was transferred to a flask, and 36 ml of water and 720 ml of dichloromethane were added. A 5% Na₃PO₄ solution was added to the biphasic system until pH 6-7 was reached. The phases were separated, and the aqueous phase was re-extracted with 360 ml of dichloromethane. The organic phases were combined and washed with 360 ml of water. All volatile components were removed from the organic phase by vacuum distillation. A solid residue of the compound of formula V (1.70 g (91.4%)) was obtained.

## Claims

1. A process for preparing finerenone, a compound of formula I 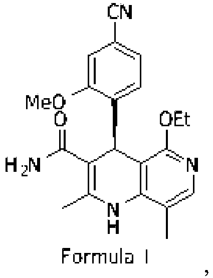 which comprises the step:
a) mixing a compound of formula II 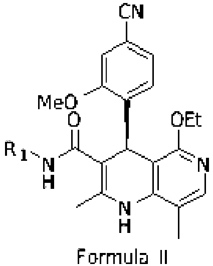 wherein
R1 is selected from
(i) C₁-C₆ alkyl,
(ii) phenyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group, and
(ii) benzyl, unsubstituted or substituted with 1 to 4 substituents independently of one another selected from halogen, methoxy, ethoxy, hydroxyl, nitro, cyano, methyl, ethyl, propyl, butyl, phenyl, or benzyl group;
with trifluoroacetic acid and/or trifluoromethanesulfonic acid.

2. The process according to claim 1, wherein in step a) the compound of formula II is mixed with both, trifluoroacetic acid and trifluoromethanesulfonic acid.

3. The process according to claim 1 or 2, which additionally comprises the steps:
b) heating the reaction mixture obtained in step a), preferably to a temperature from 40 °C to 75 °C, preferably from 45 °C to 70 °C, more preferably from 50 °C to 65 °C, and most preferably from 50 °C to 60 °C;
c) adding an antisolvent; and
d) cooling, preferably to a temperature from 0 °C to 10 °C, preferably to 5 °C, to obtain a precipitate.

4. The process according to claim 3, which additionally comprises the steps:
e) filtering off the precipitate obtained in step d);
f) dissolving or suspending the precipitate obtained in step e) in a solvent;
g) cooling, preferably to a temperature from 0 °C to 10 °C, preferably to 5 °C;
h) adjusting the pH to 8 to 10, preferably to 8 to 9, to obtain a precipitate;
i) optionally, filtering off the precipitate obtained in step h); and
j) optionally, recrystallizing the compound of formula I from a C₁-C₆ alcohol, preferably ethanol.

5. The process according to any of the preceding claims, wherein R1 is selected from the group consisting of tert-butyl, phenyl, p-methoxybenzyl, 2,4-dimethoxybenzyl, or 3,4-dimethylbenzyl.

6. The process according to any of the preceding claims, wherein the compound of formula II is a compound of formula II-a

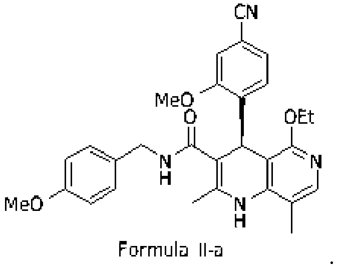

7. The process according to any of the preceding claims, wherein in the step a) no solvent is added.

8. The process according to any of the preceding claims, wherein the molar ratio of the compound of formula II or the compound of formula II-a to trifluoromethanesulfonic acid is from 1:0.2 to 1:3, preferably from 1:0.2 to 1:2, more preferably from 1:0.3 to 1:1.5, even more preferably from 1:0.3 to 1:1, and most preferably about 1:0.5.

9. The process according to any of the preceding claims, wherein the molar ratio of the compound of formula II or the compound of formula II-a to trifluoroacetic acid is from 1:10 to 1:30, preferably from 1:15 to 1:25, more preferably about 1:20.

10. The process according to any of claims 3 to 9, wherein the antisolvent added in step c) is selected from C₁-C₆ alcohols, ethyl acetate, or mixtures thereof, preferably a mixture of ethanol and ethyl acetate.

11. The process according to any of claims 4 to 10, wherein the solvent used in step f) is a mixture of a C₁-C₆ alcohol and water, more preferably a mixture of methanol or ethanol and water, and most preferably a mixture of methanol and water.

12. Finerenone hemihydrate.

13. The finerenone hemihydrate according to claim 12, which is crystalline.

14. The finerenone hemihydrate according to claim 12 or 13, which is **characterized by** an X-ray powder diffraction (XRPD) pattern having at least 5 characteristic peaks selected from 4.3°±0.2°, 7.2°±0.2°, 8.6°±0.2°, 11.4°±0.2°, 12.1°±0.2°, 17.4°±0.2°, 22.4°±0.2°, and 26.2°±0.2° 2Theta.

15. A process for preparing a compound of formula V 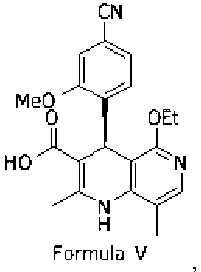 which comprises the following steps:
(i) adding a compound of formula VI to a compound of formula VII to form a salt of formula VIII 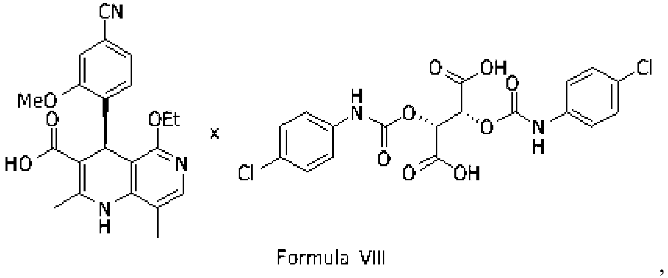
(ii) filtering off the precipitate obtained, and
(iii) neutralizing the salt by adding a base to obtain the compound of formula V.
